(19) Europäisches Patentamt
European Patent Office
Office européen des brevets

(11) **EP 4 327 737 A1**

(12) **EUROPEAN PATENT APPLICATION**
published in accordance with Art. 153(4) EPC

(43) Date of publication:
**28.02.2024 Bulletin 2024/09**

(51) International Patent Classification (IPC):
**A61B 5/08** (2006.01)

(21) Application number: **21937694.4**

(22) Date of filing: **26.11.2021**

(86) International application number:
**PCT/CN2021/133436**

(87) International publication number:
**WO 2022/222472 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.04.2021 CN 202110419179**

(71) Applicant: **Beijing Choice Electronic Technology Co., Ltd.**
**Beijing 100041 (CN)**

(72) Inventors:
• **MA, Chuanlong**
  **Beijing 100041 (CN)**
• **CHEN, Qiaoyan**
  **Beijing 100041 (CN)**

(74) Representative: **Fuchs Patentanwälte Partnerschaft mbB**
**Tower 185**
**Friedrich-Ebert-Anlage 35-37**
**60327 Frankfurt am Main (DE)**

(54) **RESPIRATORY RATE MEASUREMENT METHOD AND APPARATUS, AND ELECTRONIC DEVICE AND READABLE MEDIUM**

(57)     Provided are a method and device for measuring a respiratory rate, an electronic device, and a readable medium, which belong to the technical field of respiratory rate monitoring. The method for measuring a respiratory rate includes: acquiring a PPG signal; performing a preprocess on the PPG signal; acquiring PPG interval data and a PPG interval variation valve according to a pretreated PPG signal, wherein the PPG interval variation value refers to a difference between two adjacent PPG intervals in the PPG interval data; when there is no the abnormal PPG interval variation value in PPG interval data, calculating the respiratory rate according to the PPG interval data; and when there is the abnormal PPG interval variation value in PPG interval data, correcting the PPG interval data, and calculating the respiratory rate according to corrected PPG interval data. Therefore, the accuracy of measurement of a PPG interval variation can be improved, thereby reducing a measurement error of the respiratory rate.

Fig. 1

## Description

## Technical Field

The disclosure relates to the technical field of respiratory rate monitoring, and in particular to a method and device for measuring a respiratory rate, an electronic device, and a readable medium.

## Background

As a significant physiological parameter, the respiratory rate is the auxiliary means to determine the physical condition. Theoretically, the photoplethysmography (PPG), a common method for measuring a respiratory rate, is to acquire a PPG signal according to the light brightness change on the skin surface after blood absorbs and reflects the light, and then to analyze the PPG signal, so as to obtain the respiratory rate. However, the PPG signal tends to be interfered by a current signal, a power frequency signal, an electromagnetic signal, etc. of ambient light or dark light, resulting in inaccurate analysis result of the PPG signal, thus influencing the accuracy of the measurement of the respiratory rate.

## Summary

The disclosure provides a method and device for measuring a respiratory rate, an electronic device, and a readable medium, so as to improve the accuracy of the measurement of the respiratory rate.

In a first aspect, the disclosure provides a method for measuring a respiratory rate. The method includes:

a PPG signal is acquired;

a preprocess is performed on the PPG signal;

PPG interval data are acquired according to a pretreated PPG signal;

whether there is an abnormal PPG interval variation value in the PPG interval data is determined, wherein the PPG interval variation value refers to a difference between two adjacent PPG intervals in the PPG interval data;

when there is no the abnormal PPG interval variation value in PPG interval data, the respiratory rate is calculated according to the PPG interval data; and

when there is the abnormal PPG interval variation value in PPG interval data, the PPG interval data is corrected, and the respiratory rate is calculated according to corrected PPG interval data.

The preprocess is performed on the PPG signal includes:

a filtering process is performed on the PPG signal to remove a baseline drift and an electromyographic noise.

The filtering process is performed on the PPG signal through a Butterworth filter.

The PPG interval data is corrected includes: the abnormal PPG interval variation value in the PPG interval data is corrected through a fuzzy algorithm to obtain the corrected PPG interval data.

The abnormal PPG interval variation value in the PPG interval data is corrected through a fuzzy algorithm to obtain the corrected PPG interval data includes:

an input variable and an output variable are selected according to the PPG interval data;

a fuzzification process is performed on the input variable to obtain an input variable fuzzy set and an input variable membership function;

a fuzzification process is performed on the output variable to obtain an output variable fuzzy set and an output variable membership function;

a fuzzy rule between the input variable and the output variable is acquired;

a fuzzy set operation is performed according to the fuzzy rule to obtain a fuzzy relation set;

a fuzzy value of the output variable is acquired according to the fuzzy relation set; and

a de-fuzzification calculation is performed on the fuzzy value of the output variable to obtain the corrected PPG interval data.

The input variables are the abnormal PPG interval variation value $\triangle PNT_{md}$, a previous adjacent PPG interval variation value $\triangle PNT_{fr}$ before the abnormal PPG interval variation value, and a following adjacent PPG interval variation value $\triangle PNT_{hd}$ after the abnormal PPG interval variation value in the PPG interval data; and the output variable is a corrected abnormal PPG interval variation value $\triangle PNT'_{md}$.

The fuzzy rule is that when a first fuzzy subset, a second fuzzy subset, and a third fuzzy subset are true, there is a fourth fuzzy subset, wherein the first fuzzy subset is a fuzzy subset of the abnormal PPG interval variation value $\triangle PNT_{md}$, the second fuzzy subset is a fuzzy subset of the previous PPG interval variation value $\triangle PNT_{fr}$, the third fuzzy subset is a fuzzy subset of the following PPG interval variation value $\triangle PNT_{hd}$, and the fourth fuzzy subset is a fuzzy subset of the corrected abnormal PPG interval variation value $\triangle PNT'_{md}$.

The a fuzzy set operation is performed according to the fuzzy rule to obtain a fuzzy relation set includes:

operations are performed on corresponding elements in the first fuzzy subset, the second fuzzy subset, the third fuzzy subset, and the fourth fuzzy subset, respectively according to the fuzzy rule to determine a fuzzy relation subset; and

a union process is performed on the fuzzy relation subsets to obtain the fuzzy relation set.

The a de-fuzzification calculation is performed on the fuzzy value of the output variable through a coefficient weighted average method.

The input variable fuzzy set includes a negative large fuzzy set, a negative small fuzzy set, a zero fuzzy set, a positive small fuzzy set, and a positive large fuzzy set; and the output variable fuzzy set includes a significantly-increased fuzzy set, an approximate zero fuzzy set, and a significantly-decreased fuzzy set.

The respiratory rate is calculated according to the PPG interval data/corrected PPG interval data includes:

a peak point and a trough point are extracted from the PPG interval data/corrected PPG interval data;

PPG interval variation values are deleted , of which the sum of absolute values is less than a preset threshold, between the peak point and the trough point value adjacent to each other;

a plurality of adjacent peak points are extracted from the PPG interval data/corrected PPG interval data;

a time difference between a first peak point and a last peak point is acquired among the plurality of adjacent peak points; and

the respiratory rate is acquired according to the number of the adjacent peak points and the time difference.

In a second aspect, the disclosure provides a device for measuring a respiratory rate. The device includes: a first acquisition component configured to acquire a PPG signal;

a preprocess component configured to perform a preprocess on the PPG signal;

a second acquisition component configured to acquire PPG interval data according to a pretreated PPG signal;

a determination component configured to determine whether there is an abnormal PPG interval variation value in the PPG interval data, wherein the PPG interval variation value refers to a difference between two PPG adjacent intervals in the PPG interval data;

a calculation component configured to calculate the respiratory rate according to the PPG interval data, when there is no the abnormal PPG interval variation value in PPG interval data; and

a correction component configured to correct the PPG interval data, when there is the abnormal PPG interval variation value in PPG interval data; where

the calculation component is further configured to calculate the respiratory rate according to corrected PPG interval data.

In a third aspect, the disclosure provides an electronic device. The apparatus includes:

at least one processor; and

a memory in communication connection with the at least one processor; where

the memory stores an instruction executable by the at least one processor, and the instruction causes the at least one processor to execute any one of the methods for measuring a respiratory rate when executed by the at least one processor.

In a fourth aspect, the disclosure provides a non-transitory computer-readable storage medium, storing a computer instruction, wherein the computer instruction is configured to cause a computer to execute any one of the methods for measuring a respiratory rate.

According to the method for measuring a respiratory rate provided by the disclosure, the preprocess is performed on the PPG signal; then the PPG interval data are acquired according to the treated PPG signal; whether there is the abnormal PPG interval variation value in the PPG interval data is determined; when there is no the abnormal PPG interval variation value in PPG interval data, the respiratory rate is acquired according to the PPG interval data; when there is the abnormal PPG interval variation value in PPG interval data, the PPG interval data are corrected; and the respiratory rate is calculated according to the corrected PPG interval data. Therefore, the accuracy of measurement of a PPG interval variation may be improved, thereby reducing a measurement error of the respiratory rate.

It should be understood that the contents described in this section are not intended to identify keys or critical features of examples of the disclosure or to limit the scope of the disclosure. Other features of the disclosure will become easy to understand from the following description.

**Brief Description of the Drawings**

The accompanying drawings provide a further understanding of the disclosure as a constituent part of the description and illustrate the disclosure with examples of the disclosure, and are not intended to limit the disclosure. The above and other features and advantages will become more apparent to those skilled in the art by describing the illustrative examples in detail with reference to the accompanying drawings.

Fig. 1 is a flowchart of a method for measuring the respiratory rate according to an example of the disclosure;

Fig. 2 is a PPG interval curve graph acquired through filtered PPG interval data and a $CO_2$ concentration variation curve graph in an example of the disclosure;

Fig. 3 is a flowchart of calculating respiratory rate according to PPG interval data according to an example of the disclosure;

Fig. 4 is a flowchart of correcting PPG interval data according to an example of the disclosure;

Fig. 5 is a PPG interval curve graph acquired after correcting abnormal PPG interval data;

Fig. 6 is a flowchart of calculating respiratory rate according to corrected PPG interval data;

Fig. 7 is a block diagram of a device for measuring the respiratory rate according to an example of the disclosure; and

Fig. 8 is a block diagram of an electronic device for implementing a method for measuring the respiratory rate according to an example of the disclosure.

In the accompanying drawings:
700-device for measuring a respiratory rate; 701-first acquisition component; 702-preprocess component; 703-second acquisition component; 704-determination component; 705-calculation component; 706-correction component; 800-apparatus; 801-calculation unit; 802-read-only memory (ROM); 803-random access memory (RAM); 804-bus; 805-input/output (I/0) interface; 806-input unit; 807-output unit; 808-storage unit; and 809-communication unit.

## Detailed Description of the Embodiments

In order that those skilled in the art can better understand the technical solutions of the disclosure, the description of the illustrative examples (including various details thereof) of the disclosure is provided below in conjunction with the accompanying drawings, so as to facilitate understanding, and should be deemed as illustrative only. Therefore, those of ordinary skill in the art should realize that various changes and modifications can be made to the examples described herein without departing from the scope and spirit of the disclosure. Similarly, well-known functions and structures are not repeated in the following description for clarity and conciseness.

All the examples of the disclosure and features in the examples can be combined mutually without conflict.

As used herein, the term "and/or" includes one or any and all possible combinations of a plurality of associated items listed.

The terms used in the disclosure are merely to describe the specific examples, instead of limiting the disclosure. As used herein, the singular forms "a" and "the" are also intended to include the plural forms, unless clearly indicated otherwise in the context. It is also to be understood that the terms "comprises" and/or "made from" when used in the description specify the presence of features, integers, steps, operations, elements, and/or assemblies, but do not preclude the presence or addition of one or more other features, integers, steps, operations, elements, components, and/or groups thereof. "Connection" or "connected", etc. are not limited to physical or mechanical connections, but can include direct or indirect electrical connections.

Unless defined otherwise, all the terms (including technical and scientific terms) used herein have the same meaning as commonly understood by those of ordinary skill in the art. It is also to be understood that the terms, such as those defined in commonly used dictionaries, should be interpreted as having the same meaning as in the context of the relevant art and the disclosure, instead of the idealized or overly-formal meaning unless clearly defined herein.

The heart rate is changed with respiration. The heart rate is increased during inspiration, so as to make an RR interval (a time between R waves of two QRS waves) and a photoplethysmography (PPG) interval small. The PPG interval is prolonged during expiration. According to examples of the disclosure, respiratory rate is measured through a rule that the PPG interval is changed with a respiratory cycle.

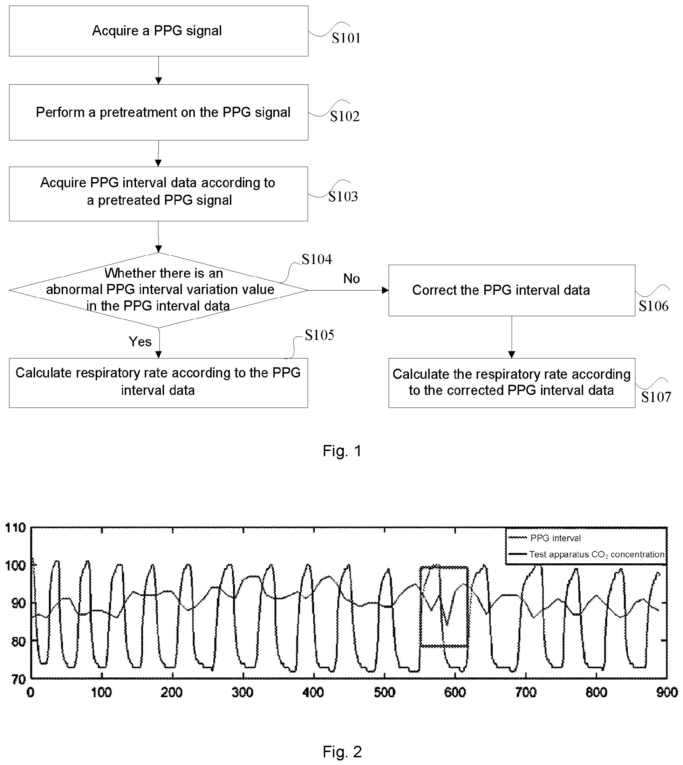

Fig. 1 is a flowchart of a method for measuring the respiratory rate according to an example of the disclosure. As shown in Fig. 1, the method for measuring a respiratory rate includes:
Step S101, a PPG signal is acquired.

The PPG signal may be acquired through a collection component, such as a thermal imaging sensor, which is not limited in the disclosure.

Step S102, a preprocess is performed on the PPG signal.

The preprocess is performed on the PPG signal to remove a baseline drift and an electromyographic noise, thereby accuracy of respiratory rate monitoring is improved.

In some examples, a filtering process is performed on the PPG signal to remove the baseline drift and the electromyographic noise from the PPG signal.

For example, a preprocess is performed on the PPG signal through a Butterworth filter. A principle of the Butterworth filter is shown in formula (1).

$$y(n) = \sum_{m=1}^{N} a_m y(n-m) + \sum_{m=0}^{M} b_m x(n-m) \qquad (1)$$

In formula (1), $a_m$ and $b_m$ are filter coefficients calculated through matlab, x is an input signal to be pretreated, and y is an output signal to be pretreated.

Step S103, PPG interval data are acquired according to a pretreated PPG signal.

After the preprocess is performed on the PPG signal, PPG data features are extracted from the pretreated PPG signal to acquire the PPG interval data. A PPG interval curve and a PPG interval variation value may be acquired according to the PPG interval data. The PPG interval curve is configured to present the PPG interval data, so that a user may intuitively know the trend of the PPG interval data through the PPG interval curve.

In some examples, a $CO_2$ concentration is acquired through a test apparatus while the PPG signal is acquired, and then a $CO_2$ concentration variation curve and the PPG interval curve are placed in the same coordinate system, so as to compare PPG intervals conveniently.

Fig. 2 is a PPG interval curve graph acquired according to filtered PPG interval data, and a $CO_2$ concentration variation curve graph. The abscissas represent the number of sampling points, and the ordinates represent the $CO_2$ concentration and the PPG interval.

In Fig. 2, an increase in $CO_2$ concentration indicates an expiratory process, and a decrease in $CO_2$ concentration indicates an inspiratory process. A variation of the $CO_2$ concentration measured by a $CO_2$ concentration measurement apparatus is not completely synchronous with a respiratory process, and the PPG interval and the RR interval also have lags. However, the PPG interval variation may still reflect a PPG interval variation rule caused by respiration.

Step S104, whether there is an abnormal PPG interval variation value in the PPG interval data is determined.

The PPG interval variation value refers to a difference between two adjacent PPG intervals in the PPG interval data.

The PPG interval is varied under the influence of physiological, external interference or signal processs, etc. which influences calculation of a final respiratory rate. In order to improve the accuracy and stability of measurement, the PPG interval variation value is acquired from the PPG interval data. When there is no the abnormal PPG interval variation value in the PPG interval data, the PPG interval data are directly utilized to calculate the respiratory rate. When there is the abnormal PPG interval variation value in PPG interval data, the PPG interval data are required to be corrected first, and then the respiratory rate is calculated according to corrected PPG interval data.

In some examples, whether there is the abnormal PPG interval variation value in the PPG interval data is determined intuitively through the PPG interval curve. As shown in Fig. 2, a PPG interval variation value in a box is abnormal, and a PPG interval in the middle is prolonged slightly, while adjacent PPG intervals before and after are shortened greatly.

It should be noted that the method for determining whether there is the abnormal PPG interval variation value is not limited to the PPG interval curve, and it may also be acquired by analyzing the PPG interval data. The method for determining whether there is an abnormal PPG interval variation value is not limited in the disclosure.

Step S105, when there is no the abnormal PPG interval variation value in PPG interval data, the respiratory rate is calculated according to the PPG interval data.

Step S106, when there is the abnormal PPG interval variation value in PPG interval data, the PPG interval data are corrected.

Step S107, the respiratory rate is calculated according to corrected PPG interval data.

When there is no the abnormal PPG interval variation value in PPG interval data, the respiratory rate is calculated according to the PPG interval data directly.

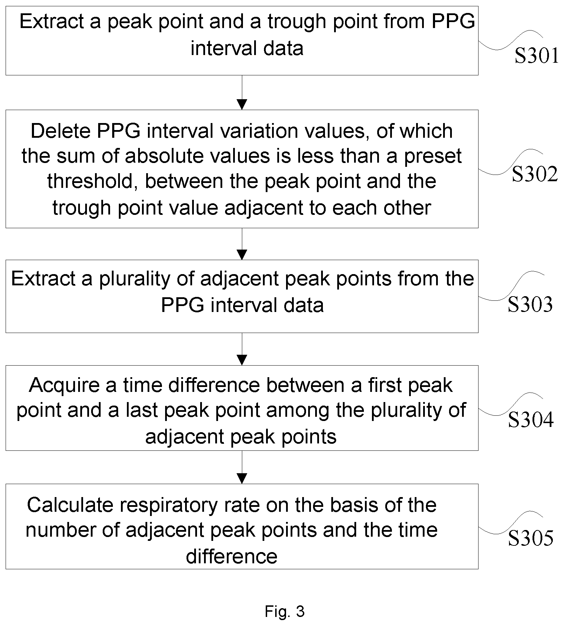

As shown in Fig. 3, the step that the respiratory rate is calculated according to the PPG interval data includes:

Step S301, a peak point and a trough point are extracted from the PPG interval data.

By analyzing the PPG interval data, the peak point and the trough point are extracted from the PPG interval data, wherein the peak point and the trough point correspond to a peak and a trough in the PPG interval curve, respectively. The peak point and the trough point are a maximum (vertex) and a minimum (lowest point) of all PPG interval variation values in the PPG interval curve, respectively. A PPG interval variation value on the left side of the peak point increases consistently, which indicates the expiratory process. A PPG interval variation value on the right side of the peak point decreases consistently, which indicates the inspiratory process.

It should be noted that when the PPG interval variation value is zero, only one PPG interval variation value is extracted.

S302, PPG interval variation values, of which the sum of absolute values is less than a preset threshold, between the peak point and the trough point value adjacent to each other are deleted.

A too small PPG interval variation is caused by a measurement error or respiration not in a strict sense, so that the sum of the absolute values of the PPG interval variation values between the peak point and the trough point adjacent to each other is calculated. When the sum of the absolute values is less than the preset threshold, the PPG interval variation values are deleted. The preset threshold is set according to the specific situation in the art, which is not limited in the disclosure.

Step S303, a plurality of adjacent peak points are extracted from the PPG interval data.

In some examples, n adjacent peak points are extracted from the PPG interval data, wherein n is a positive integer greater than 2. It should be noted that the PPG interval data in step S303 refer to PPG interval data acquired after deleting the PPG interval variation values, of which the sum of the absolute values is less than the preset threshold, between the peak point and the trough point adjacent to each other.

Step S304, a time difference between a first peak point and a last peak point among the plurality of adjacent peak points is acquired.

Since the peak point is arranged in a measurement time order, a sampling time may be acquired through the time difference between the peak points. After the first peak point and the last peak point among the plurality of adjacent peak points are acquired, the time difference may be determined according to sampling times corresponding to the first peak point and the last peak point.

Step S305, the respiratory rate is calculated according to the number of adjacent peak points and the time difference.

The respiratory rate is a number of respirators in a set time, for example, a number of respirators in 60s.

For example, the respiratory rate = $(n-1) \times (60s/t)$.

In some examples, when there is the abnormal PPG interval variation value in PPG interval data, the PPG interval data are corrected first, and then the respiratory rate is calculated according to corrected PPG interval data.

The step that the PPG interval data are corrected includes: the abnormal PPG interval variation value in the PPG interval data is corrected through a fuzzy algorithm to obtain the corrected PPG interval data.

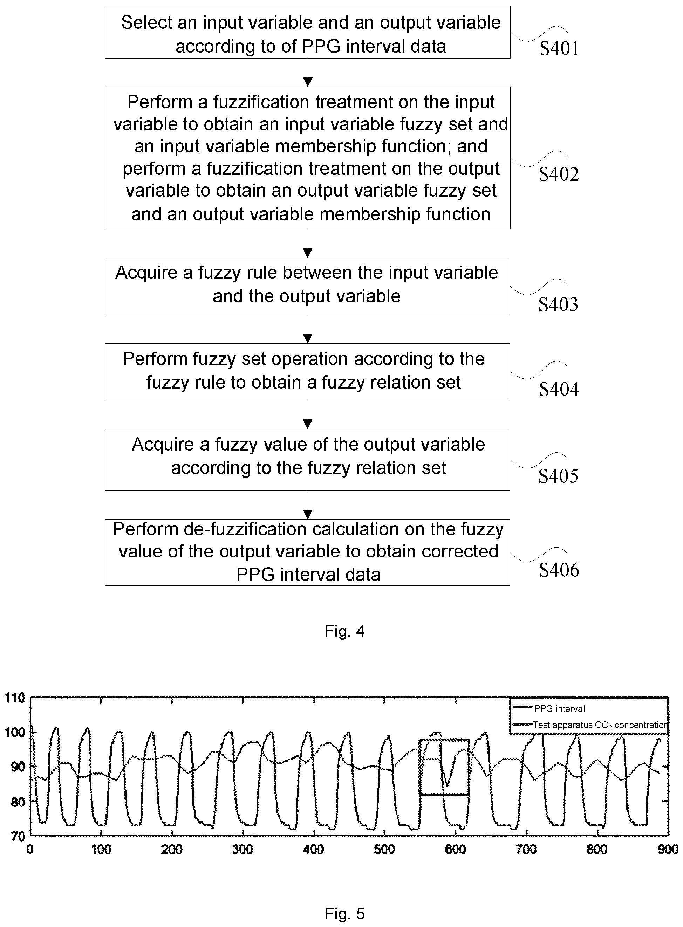

Fig. 4 is a flowchart of correcting PPG interval data according to an example of the disclosure. As shown in Fig. 4, the step that the abnormal PPG interval variation value in the PPG interval data is corrected through a fuzzy algorithm to obtain the corrected PPG interval data includes:

Step S401, an input variable and an output variable are selected according to the PPG interval data.

The input variable and the output variable for the fuzzy algorithm are selected from the PPG interval data. In some examples, the input variables are an abnormal PPG interval variation value $\triangle PNT_{md}$, a previous adjacent PPG interval variation value $\triangle PNT_{fr}$ before the abnormal PPG interval variation value, and a following adjacent PPG interval variation value $\triangle PNT_{hd}$ after the abnormal PPG interval variation value in the PPG interval data. A corrected abnormal PPG interval variation value $\triangle PNT'_{md}$ is used as the output variable.

Step S402, a fuzzification process is performed on the input variable to obtain an input variable fuzzy set and an input variable membership function; and a fuzzification process is performed on the output variable to obtain an output variable fuzzy set and an output variable membership function.

In some examples, the input variable fuzzy set may be divided into five input variable fuzzy sets as required, and the output variable fuzzy set may be divided into three output variable fuzzy sets as required.

For example, the input fuzzy sets include a negative large fuzzy set, a negative small fuzzy set, a zero fuzzy set, a positive small fuzzy set, and a positive large fuzzy set. The negative large fuzzy set means that a first difference between each input variable in the fuzzy set and a first preset intermediate value is large, and the first difference is negative, that is, the input variable is less than the first intermediate value. The negative small fuzzy set means that a second difference between each input variable in the fuzzy set and the first preset intermediate value is small, and the second difference is negative, that is, the input variable is less than the first intermediate value, and an absolute value of the second difference is greater than that of the first difference. The zero fuzzy set means that a difference between each input variable in the fuzzy set and the first preset intermediate value is zero. The positive small fuzzy set means that a third difference between each input variable in the fuzzy set and a first preset intermediate value is small, and the third difference value is positive, that is, the input variable is greater the first intermediate value. The positive large fuzzy set means that a fourth difference between each input variable in the fuzzy set and the first preset intermediate value is large, and the fourth difference is positive, that is, the input variable is greater than the first intermediate value, and an absolute value of the fourth difference is greater than that of the third difference.

The output variable fuzzy sets include a significantly-increased fuzzy set, an approximate zero fuzzy set, and a significantly-decreased fuzzy set. The significantly-increased fuzzy set means that each output variable in the fuzzy set is greater than a second preset intermediate value, and an absolute value of a fifth difference between the output variable and the second preset intermediate value is greater than a set threshold. In the approximate zero fuzzy set, a sixth difference between each output variable in the fuzzy set and the second preset intermediate value is small, and an absolute value of the sixth difference is less than a set threshold. The significantly-decreased fuzzy set means that each output variable in the fuzzy set is less than the second preset intermediate value, and an absolute value of a seventh difference between the output variable and the second preset intermediate value is greater than a set threshold.

In some examples, the input variable membership function is a triangular function, and the output variable membership function is a gradient function.

Step S403, a fuzzy rule between the input variable and the output variable is acquired.

The fuzzy rule is determined according to the input variable and the output variable. For example, the fuzzy rule is that if a first fuzzy subset A, a second fuzzy subset B, and a third fuzzy subset C are true, there is a fourth fuzzy subset D.

The fuzzy rule may be expressed as: if A and B and C then D.

The first fuzzy subset A is a fuzzy subset of the abnormal PPG interval variation value $\triangle PNT_{md}$, the second fuzzy subset B is a fuzzy subset of the previous PPG interval variation value $\triangle PNT_{fr}$, the third fuzzy subset C is a fuzzy subset of the following PPG interval variation value $\triangle PNT_{hd}$, and the fourth fuzzy subset D is a fuzzy subset of the corrected abnormal PPG interval variation value $\triangle PNT'_{md}$.

In some examples, by integrating the experience of a measurer into the fuzzy rule, that is, the fuzzy rule is acquired according to the experience, and the experience of the measurer is integrated into determination of the PPG interval variation, so that the accuracy of the determination of the PPG interval variation is improved.

Step S404, a fuzzy set operation is performed according to the fuzzy rule to obtain a fuzzy relation set.

The fuzzy relation set includes at least one fuzzy relation subset. A corresponding operation is performed according to the fuzzy rule to obtain the fuzzy relation subset, and a union process is performed on a plurality of fuzzy relation subsets to obtain the fuzzy relation set.

In some examples, operations are performed on corresponding elements in the first fuzzy subset, the second fuzzy subset, the third fuzzy subset, and the fourth fuzzy subset, respectively according to the fuzzy rule to determine the fuzzy relation subset. The union process is performed on the fuzzy relation subsets to obtain the fuzzy relation set.

For example, a fuzzy set operation is performed on the elements in the first fuzzy subset A, the second fuzzy subset B, the third fuzzy subset C, and the fourth fuzzy subset D according to the fuzzy rule to determine the fuzzy relation subset Ri= A×B×C×D, and then a union of the fuzzy relation subsets is solved to obtain a fuzzy relation set R, wherein R= UR$_i$.

Step S405, a fuzzy value of the output variable is acquired according to the fuzzy relation set.

In step S405, the fuzzy value of the output variable is calculated according to the fuzzy relation set, that is, the fuzzy value u of the output variable is acquired according to the previous PPG interval variation value $\triangle PNT_{fr}$, the abnormal PPG interval variation value $\triangle PNT_{md}$, the following PPG interval variation value $\triangle PNT_{hd}$, and the fuzzy relation set R.

Specifically, u= [$\triangle PNT_{fr}$, $\triangle PNT_{md}$, $\triangle PNT_{hd}$]·R.

Step S406, a de-fuzzification calculation is performed on the fuzzy value of the output variable to obtain the corrected PPG interval data.

In step S406, the a de-fuzzification calculation is performed on the fuzzy value of the output variable through a coefficient weighted average method to obtain the corrected PPG interval data.

For example, the a de-fuzzification calculation is performed through a de-fuzzification calculation formula (3).

$$\Delta PNT''_{md} = \sum k_i \bullet \Delta PNT_i \, / \, \sum k_i \quad (3)$$

In formula (3), $\triangle PNT'_{md}$ represents the corrected PPG interval variation value, $k_i$ represents an ith weighting coefficient, and $\triangle PNT_i$ represents an ith PPG interval variation value.

The process is performed on the abnormal PPG interval data through the fuzzy algorithm. If it is determined that the reduction of the PPG interval variation values at a box position in Fig. 4 is caused by interference, the abnormal PPG interval variation value is deleted to obtain the corrected PPG interval data. In order to know the corrected PPG interval data intuitively, the corrected PPG interval data are presented through a PPG interval curve graph in Fig. 5.

Fig. 5 is the PPG interval curve graph acquired after the abnormal PPG interval data are corrected. By comparing the box positions in Figs. 4 and 5, it can be seen that after the abnormal PPG interval variation values are deleted, the abnormal PPG interval variation values in a PPG interval curve are eliminated.

In the example of the disclosure, according to the fuzzy algorithm, the abnormal PPG interval variation value $\triangle PNT_{md}$ is corrected through the previous adjacent PPG interval variation value $\triangle PNT_{fr}$, positioned before the abnormal PPG interval variation value and the following adjacent PPG interval variation value $\triangle PNT_{hd}$ positioned after the abnormal PPG interval variation value. The PPG interval variation value is determined through the fuzzy rule. Therefore, the experience of the measurer may be integrated into the determination of the PPG interval variation, thereby reducing the influence, on the PPG interval variation, from other factors, and improving the accuracy of the determination of the PPG interval variation.

After the abnormal PPG interval data are corrected, the respiratory rate is calculated according to the corrected PPG interval data. The difference between the calculation of the respiratory rate according to uncorrected raw PPG interval data and the calculation of the respiratory rate according to the corrected PPG interval data lies in the basis for calculating the respiratory rate, that is, the PPG interval data employed are different, but the calculation steps and principles are the same. For a better understanding of the disclosure, the steps of calculating the respiratory rate according to the corrected PPG interval data are described below.

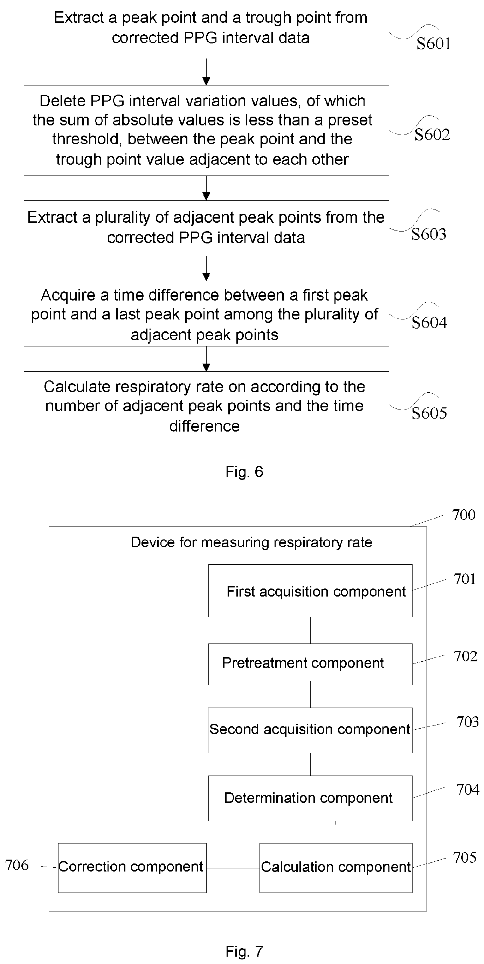

As shown in Fig. 6, the step that the respiratory rate is calculated according to the corrected PPG interval data includes:

Step S601, a peak point and a trough point are extracted from the corrected PPG interval data.

By analyzing the PPG interval data, the peak point and the trough point are extracted from the corrected PPG interval data, wherein the peak point and the trough point correspond to a peak point and a trough point in a corrected PPG interval curve, respectively. The peak point and the trough point are a maximum (vertex) and a minimum (lowest point) of all PPG interval variation values in the corrected PPG interval curve, respectively. A PPG interval variation value on the left side of the peak point increases consistently, which indicates an expiratory process. A PPG interval variation value on the right side of the peak point decreases consistently, which indicates an inspiratory process.

It should be noted that when the PPG interval variation value is zero, only one PPG interval variation value is extracted.

Step S602, PPG interval variation values, of which the sum of absolute values is less than a preset threshold, between the peak point and the trough point value adjacent to each other are deleted.

A too small PPG interval variation is caused by a measurement error or respiration not in a strict sense, so that the sum of the absolute values of the PPG interval variation values between the peak point and the trough point adjacent to each other is calculated. If the sum of the absolute values is less than the preset threshold, the PPG interval variation values are deleted. The preset threshold is set according to the specific situation in the art, which is not limited in the disclosure.

Step S603, a plurality of adjacent peak points are extracted from the corrected PPG interval data.

In some examples, n adjacent peak points are extracted from the corrected PPG interval data, wherein n is a positive integer greater than 2.

Step S604, a time difference between a first peak point and a last peak point among the plurality of adjacent peak points is acquired.

Since the peak point is arranged in a measurement time order, a sampling time may be acquired through the time difference between the peak points. After the first peak point and the last peak point among the plurality of adjacent peak points are acquired, the time difference may be determined through sampling times corresponding to the first peak point and the last peak point.

Step S605, the respiratory rate is calculated according to the number of adjacent peak points and the time difference.

The respiratory rate is a number of respiration in a set time. For example, a number of respiration in 60s. For example, the respiratory rate = (n-1) $\times$ (60s/t).

According to the method for measuring a respiratory rate provided by the disclosure, the process is performed on the PPG signal; then the PPG interval data and the PPG interval variation value are acquired according to the treated PPG signal; when there is no the abnormal PPG interval variation value in PPG interval data, the respiratory rate is acquired according to the PPG interval data; when there is the abnormal PPG interval variation value in PPG interval data, the abnormal PPG interval variation value is corrected; and the respiratory rate is calculated according to the corrected PPG interval data. Therefore, the accuracy of measurement of a PPG interval variation may be improved, thereby reducing a measurement error of the respiratory rate.

Fig. 7 is a block diagram of a device for measuring the respiratory rate according to an example of the disclosure. As shown in Fig. 7, the device 700 for measuring a respiratory rate includes:

a first acquisition component 701 configured to acquire a PPG signal, wherein the first acquisition component 701 may be a collection component, such as a thermal imaging sensor, and the thermal imaging sensor may be of a finger-clip type for facilitating wearing, which is not limited in the disclosure;

a preprocess component 702 configured to perform a preprocess on the PPG signal, wherein the preprocess is performed on the PPG signal to remove a baseline drift and an electromyographic noise, thereby improving the accuracy of respiratory rate monitoring;

a second acquisition component 703 configured to acquire PPG interval data according to a pretreated PPG signal, wherein after the preprocess is performed on the PPG signal, PPG data features are extracted from the pretreated PPG signal to acquire the PPG interval data, and a PPG interval curve and a PPG interval variation value may be acquired according to the PPG interval data, the PPG interval curve being configured to present the PPG interval data, so that a user may intuitively know the trend of the PPG interval data through the PPG interval curve;

a determination component 704 configured to determine whether there is an abnormal PPG interval variation value in the PPG interval data.

And the PPG interval variation value refers to a difference between two adjacent PPG intervals in the PPG interval data,

a PPG interval is varied under the influence of physiological, external interference or signal processs, etc. which influences calculation of a final respiratory rate; in order to improve the accuracy and stability of measurement, the PPG interval variation value is acquired from the PPG interval data; if there is no the abnormal PPG interval variation value in the PPG interval data, the PPG interval data are directly utilized to calculate the respiratory rate; and if there is the abnormal PPG interval variation value in PPG interval data, the PPG interval data are required to be corrected first, and then the respiratory rate is calculated according to corrected PPG interval data;

a calculation component 705 configured to calculate the respiratory rate according to the PPG interval data, when there is no the abnormal PPG interval variation value in PPG interval data.

And the step that the calculation component 705 calculates the respiratory rate according to the PPG interval data mainly includes: a peak point and a trough point are extracted from the PPG interval data; PPG interval variation values, of which the sum of absolute values is less than a preset threshold, between the peak point and the trough point value adjacent to each other are deleted; a plurality of adjacent peak points are extracted from the PPG interval data; a time difference between a first peak point and a last peak point among the plurality of adjacent peak points is acquired; and the respiratory rate is calculated according to the number of adjacent peak points and the time difference; and a correction component 706 configured to correct the PPG interval data, when there is the abnormal PPG interval variation value in PPG interval data .

And the correction component 706 is configured to perform following steps: an input variable and an output variable are selected according to the PPG interval data; a fuzzification process is performed on the input variable to obtain an input variable fuzzy set and an input variable membership function; a fuzzification process is performed on the output variable to obtain an output variable fuzzy set and an output variable membership function; a fuzzy rule between the input variable and the output variable is acquired; a fuzzy set operation is performed according to the fuzzy rule to obtain a fuzzy relation set; a fuzzy value of the output variable is acquired according to the fuzzy relation set; and a de-fuzzification calculation is performed on the fuzzy value of the output variable to obtain the corrected PPG interval data.

The calculation component 705 is further configured to calculate the respiratory rate according to the corrected PPG interval data.

The calculation component 705 is configured to perform following steps: a peak point and a trough point are extracted from the corrected PPG interval data; PPG interval variation values, of which the sum of absolute values is less than a preset threshold, between the peak point and the trough points value adjacent to each other are deleted; a plurality of adjacent peak points are extracted from the PPG interval data; a time difference between a first peak point and a last peak point among the plurality of adjacent peak points is acquired; and the respiratory rate is calculated according to the number of adjacent peak points and the time difference.

In some examples, the device for measuring a respiratory rate further includes a display component configured to display the respiratory rate.

The functions or components encompassed in the device according to the example of the disclosure may be used to implement the above method for measuring a respiratory rate, and the specific implementation and technical effects thereof may refer to the description of the above method example, which will not be repeated herein for brevity.

In the device for measuring the respiratory rate according to the example of the disclosure, the first acquisition component acquires the PPG signal; the preprocess component performs the process on the PPG signal; the second acquisition component acquires the PPG interval data and the PPG interval variation value according to the treated PPG signal; the calculation component calculates the respiratory rate according to the PPG interval data when there is no the abnormal PPG interval variation value in PPG interval data; the correction component corrects the PPG interval data when there is the abnormal PPG interval variation value in PPG interval data; and the calculation component calculates the respiratory rate according to the corrected PPG interval data. Therefore, the accuracy of measurement of a PPG interval variation may be improved, thereby reducing a measurement error of the respiratory rate.

The disclosure further provides an electronic device, a readable storage medium, and a computer program product.

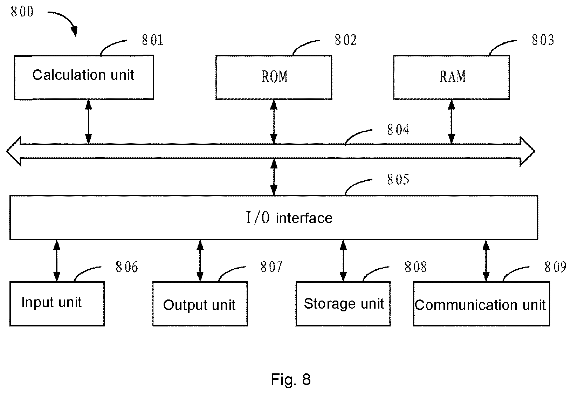

Fig. 8 is a schematic block diagram of an illustrative electronic device 800 for implementing an example of the disclosure. The electronic device is intended to represent various forms of digital computers, such as laptop computers, table computers, workstations, personal digital assistants, servers, blade servers, mainframe computers, and other suitable computers. The electronic device may also represent various forms of mobile devices, such as personal digital processing devices, cell phones, smart phones, wearable devices, and other similar calculation devices. The components illustrated herein, their connections and relations, and their functions are only illustrative, and are not intended to limit the implementation of the disclosure as described and/or claimed herein.

As shown in Fig. 8, the apparatus 800 includes a calculation unit 801 that may execute various suitable actions and processs according to a computer program stored in a read-only memory (ROM) 802 or loaded into a random access memory (RAM) 803 from a storage unit 808. The RAM 803 may also store various programs and data required for the operation of the apparatus 800. The calculation unit 801, the ROM 802, and the RAM 803 are connected to one another through a bus 804. An input/output (I/O) interface 805 is also connected to the bus 804.

A plurality of components in apparatus 800 are connected to the I/O interface 805, including: an input unit 806 such as a keyboard, a mouse, etc.; an output unit 807 such as various types of displays, speakers, etc. ; a storage unit 808 such as a magnetic disk, an optical disk, etc.; and a communication unit 809 such as a network card, a modem, a wireless communication transceiver, etc. The communication unit 809 allows the apparatus 800 to exchange information/data with other apparatuses through a computer network such as the Internet, and/or various telecommunication networks.

The calculation unit 801 may be any one of various general and/or dedicated processing assemblies having process and calculation capacities. Some instances of the calculation unit 801 include, but are not limited to, a central processing unit (CPU), a graphics processing unit (GPU), various dedicated artificial intelligence (AI) calculation chips, various calculation units running machine learning model algorithms, a digital signal processor (DSP), and any suitable processor, controller, microcontroller, etc. The calculation unit 801 executes various methods and processs described above, such as the method for measuring a respiratory rate. For example, in some examples, the method for measuring a respiratory rate may be implemented as a computer software program tangibly encompassed in a machine-readable medium, such as the storage unit 808. In some examples, some or all of the computer programs may be loaded and/or mounted on the apparatus 800 via the ROM 802 and/or the communication unit 809. When loaded into the RAM 803 and executed by the calculation unit 801, the computer program may execute one or more steps of the method for measuring a respiratory rate described above. Alternatively, in other examples, the calculation unit 801 may be configured to execute the method for measuring a respiratory rate in any other suitable modes (for example, by means of firmware).

Various embodiments of the systems and techniques described above can be implemented in a digital electronic circuit system, an integrated circuit system, a field programmable gate array (FPGA), an application specific integrated circuit (ASIC), an application specific standard product (ASSP), a system on a chip (SOC), a load programmable logic device (CPLD), computer hardware, firmware, software, and/or combinations thereof herein. These various embodiments can include: implementation in one or more computer programs executable and/or interpretable on a programmable system including at least one programmable processor. The programmable processor can be a dedicated or general programmable processor that can receive data and instructions from a storage system, at least one input device, and at least one output device, and transmit the data and the instructions to the storage system, the at least one input device, and the at least one output device.

Program codes configured to implement the method of the disclosure can be written in one or any combination of a plurality of programming languages. These program codes can be provided for a processor or controller of the general computer, the dedicated computer, or other programmable data processing devices, so that the program codex cause functions/operations specified in the flowchart and/or block diagram to be implemented when executed by the processor or controller.

The program codes can be executed on a machine in all or in part, executed on the machine in part as an independent software package, executed on a remote machine in part, or executed on the remote machine or a server in all.

In the context of the disclosure, the machine-readable medium can be a tangible medium that can encompass or store a program for use by or in combination with an instruction execution system, device, or apparatus. The machine-readable medium can be a machine-readable signal medium or a machine-readable storage medium. The machine-readable media can include, but are not limited to, electronic, magnetic, optical, electromagnetic, infrared, or semiconductor systems, devices, or apparatuses, or any suitable combination thereof. More specific instances of the machine-readable storage media would include a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage apparatus, a magnetic storage apparatus that are each electrically connected through one or more wires, or any suitable combination thereof.

In order to provide interaction with a user, the systems and techniques described herein can be implemented on a computer. The computer is provided with a display device (for example, a cathode ray tube (CRT) or a liquid crystal display (LCD) monitor) for displaying information to the user; and a keyboard and a pointing device (for example, a mouse or trackball) through which the user can provide an input for the computer. Other types of devices can also be configured to provide interaction with a user. For example, feedback provided for the user can be any form of sensory feedback (for example, visual feedback, auditory feedback, or haptic feedback). The input from the user can be received in any form, including an acoustic input, a voice input, and a haptic input.

The systems and techniques described herein can be implemented on a calculation system (for example, a data server) that includes a background component, or a calculation system (for example, an application server) that includes a middleware component, or a calculation system (for example, a user computer having a graphical user interface or a web browser through which the user can interact with the embodiments of the systems and techniques described herein) that includes a front-end component, or a calculation system including any combination of the background component, the middleware component, or the front-end component. The components of the system can be interconnected through digital data communication (for example, a communication network) in any form or medium. Instances of the communication network include: a local area network (LAN), a wide area network (WAN), and the Internet.

The computer system can include a client and a server. Generally, the client and the server are away from each other and interact with each other through the communication network. The relation between the client and the server is generated through the computer programs running on corresponding computers and being in a client-server relation.

According to an example of the disclosure, the disclosure further provides a computer program product. The computer program product includes a computer program, wherein the computer program implements any one of the methods for measuring a respiratory rate described above when executed by a processor.

It should be understood that various forms of the flows illustrated above can be used, with steps re-ranked, added, or deleted. For example, various steps described in the disclosure can be executed in parallel, in sequence, or in different orders, as long as the desired results of the technical solutions disclosed in the disclosure can be realized, which is not limited herein.

The particular embodiments described above are not intended to limit the scope of protection of the disclosure. Those skilled in the art should understand that various modifications, combinations, subcombinations, and substitutions can be made according to the design requirements, etc. Any modifications, equivalent replacements, improvements, etc. made within the spirit and the principles of the disclosure should all fall within the scope of protection of the disclosure.

## Claims

1. A method for measuring a respiratory rate, comprising:

   acquiring a photoplethysmography (PPG) signal;
   performing a preprocess on the PPG signal;
   acquiring PPG interval data according to a pretreated PPG signal;
   determining whether there is an abnormal PPG interval variation value in the PPG interval data, wherein the PPG interval variation value refers to a difference between two adjacent PPG intervals in the PPG interval data;
   when there is no the abnormal PPG interval variation value in PPG interval data, calculating the respiratory rate according to the PPG interval data; and
   when there is the abnormal PPG interval variation value in PPG interval data, correcting the PPG interval data, and calculating the respiratory rate according to corrected PPG interval data.

2. The method for measuring the respiratory rate according to claim 1, wherein the performing a preprocess on the PPG signal comprises:
   performing a filtering process on the PPG signal to remove a baseline drift and an electromyographic noise.

3. The method for measuring the respiratory rate according to claim 2, wherein the filtering process is performed on the PPG signal through a Butterworth filter.

4. The method for measuring the respiratory rate according to claim 1, wherein the correcting the PPG interval data comprises:
   correcting the abnormal PPG interval variation value in the PPG interval data through a fuzzy algorithm to obtain the corrected PPG interval data.

5. The method for measuring the respiratory rate according to claim 4, wherein the correcting the abnormal PPG interval variation value in the PPG interval data through a fuzzy algorithm to obtain the corrected PPG interval data comprises:

   selecting an input variable and an output variable according to the PPG interval data;
   performing a fuzzification process on the input variable to obtain an input variable fuzzy set and an input variable membership function;
   performing a fuzzification process on the output variable to obtain an output variable fuzzy set and an output variable membership function;
   acquiring a fuzzy rule between the input variable and the output variable;
   performing a fuzzy set operation according to the fuzzy rule to obtain a fuzzy relation set;
   acquiring a fuzzy value of the output variable according to the fuzzy relation set; and
   performing a de-fuzzification calculation on the fuzzy value of the output variable to obtain the corrected PPG interval data.

6. The method for measuring the respiratory rate according to claim 5, wherein the input variables are the abnormal PPG interval variation value $\triangle PNT_{md}$, a previous adjacent PPG interval variation value $\triangle PNT_{fr}$ before the abnormal PPG interval variation value, and a following adjacent PPG interval variation value $\triangle PNT_{hd}$ after the abnormal PPG interval variation value in the PPG interval data; and
   the output variable is a corrected abnormal PPG interval variation value $\triangle PNT'_{md}$.

7. The method for measuring the respiratory rate according to claim 5, wherein the fuzzy rule is that when a first fuzzy subset, a second fuzzy subset, and a third fuzzy subset are true, there is a fourth fuzzy subset, wherein the first fuzzy subset is a fuzzy subset of the abnormal PPG interval variation value $\triangle PNT_{md}$, the second fuzzy subset is a fuzzy subset of the previous PPG interval variation value $\triangle PNT_{fr}$,

the third fuzzy subset is a fuzzy subset of the following PPG interval variation value $\triangle PNT_{hd}$, and the fourth fuzzy subset is a fuzzy subset of the corrected abnormal PPG interval variation value $\triangle PNT'_{md}$.

8. The method for measuring the respiratory rate according to claim 7, wherein the performing a fuzzy set operation according to the fuzzy rule to obtain a fuzzy relation set comprises:

performing operations on corresponding elements in the first fuzzy subset, the second fuzzy subset, the third fuzzy subset, and the fourth fuzzy subset, respectively according to the fuzzy rule to determine a fuzzy relation subset; and performing a union process on the fuzzy relation subsets to obtain the fuzzy relation set.

9. The method for measuring the respiratory rate according to claim 5, wherein the a de-fuzzification calculation is performed on the fuzzy value of the output variable through a coefficient weighted average method.

10. The method for measuring the respiratory rate according to any one of claims 5-9, wherein the input variable fuzzy set comprises a negative large fuzzy set, a negative small fuzzy set, a zero fuzzy set, a positive small fuzzy set, and a positive large fuzzy set; and the output variable fuzzy set comprises a significantly-increased fuzzy set, an approximate zero fuzzy set, and a significantly-decreased fuzzy set.

11. The method for measuring the respiratory rate according to any one of claims 1-9, wherein the calculating the respiratory rate according to the PPG interval data/corrected PPG interval data comprises:

extracting a peak point and a trough point from the PPG interval data/corrected PPG interval data;
deleting PPG interval variation values, of which the sum of absolute values is less than a preset threshold, between the peak point and the trough point value adjacent to each other;
extracting a plurality of adjacent peak points from the PPG interval data/corrected PPG interval data;
acquiring a time difference between a first peak point and a last peak point among the plurality of adjacent peak points; and
acquiring the respiratory rate according to the number of the adjacent peak points and the time difference.

12. A device for measuring a respiratory rate, comprising:

a first acquisition component configured to acquire a PPG signal;
a preprocess component configured to perform a preprocess on the PPG signal;
a second acquisition component configured to acquire PPG interval data according to a pretreated PPG signal;
a determination component configured to determine whether there is an abnormal PPG interval variation value in the PPG interval data, wherein the PPG interval variation value refers to a difference between two PPG adjacent intervals in the PPG interval data;
a calculation component configured to calculate the respiratory rate according to the PPG interval data, when there is no the abnormal PPG interval variation value in PPG interval data; and
a correction component configured to correct the PPG interval data, when there is the abnormal PPG interval variation value in PPG interval data; wherein
the calculation component is further configured to calculate the respiratory rate according to corrected PPG interval data.

13. An electronic device, comprising:

at least one processor; and
a memory in communication connection with the at least one processor; wherein
the memory stores an instruction executable by the at least one processor, and the instruction causes the at least one processor to execute the method according to any one of claims 1-11 when executed by the at least one processor.

14. A non-transitory computer-readable storage medium, storing a computer instruction, wherein the computer instruction is configured to cause a computer to execute the method according to any one of claims 1-11.

Acquire a PPG signal     S101

Perform a pretreatment on the PPG signal     S102

Acquire PPG interval data according to a pretreated PPG signal     S103

S104 Whether there is an abnormal PPG interval variation value in the PPG interval data

No → Correct the PPG interval data     S106

Yes     S105

Calculate respiratory rate according to the PPG interval data

Calculate the respiratory rate according to the corrected PPG interval data     S107

Fig. 1

Fig. 2

Extract a peak point and a trough point from PPG interval data ⌇ S301

↓

Delete PPG interval variation values, of which the sum of absolute values is less than a preset threshold, between the peak point and the trough point value adjacent to each other ⌇ S302

↓

Extract a plurality of adjacent peak points from the PPG interval data ⌇ S303

↓

Acquire a time difference between a first peak point and a last peak point among the plurality of adjacent peak points ⌇ S304

↓

Calculate respiratory rate on the basis of the number of adjacent peak points and the time difference ⌇ S305

Fig. 3

Select an input variable and an output variable according to of PPG interval data — S401

Perform a fuzzification treatment on the input variable to obtain an input variable fuzzy set and an input variable membership function; and perform a fuzzification treatment on the output variable to obtain an output variable fuzzy set and an output variable membership function — S402

Acquire a fuzzy rule between the input variable and the output variable — S403

Perform fuzzy set operation according to the fuzzy rule to obtain a fuzzy relation set — S404

Acquire a fuzzy value of the output variable according to the fuzzy relation set — S405

Perform de-fuzzification calculation on the fuzzy value of the output variable to obtain corrected PPG interval data — S406

Fig. 4

Fig. 5

Extract a peak point and a trough point from corrected PPG interval data — S601

Delete PPG interval variation values, of which the sum of absolute values is less than a preset threshold, between the peak point and the trough point value adjacent to each other — S602

Extract a plurality of adjacent peak points from the corrected PPG interval data — S603

Acquire a time difference between a first peak point and a last peak point among the plurality of adjacent peak points — S604

Calculate respiratory rate on according to the number of adjacent peak points and the time difference — S605

Fig. 6

700

Device for measuring respiratory rate

First acquisition component — 701

Pretreatment component — 702

Second acquisition component — 703

Determination component — 704

706 — Correction component

Calculation component — 705

Fig. 7

800

801 Calculation unit

802 ROM

803 RAM

804

805 I/O interface

806 Input unit

807 Output unit

808 Storage unit

809 Communication unit

Fig. 8

## INTERNATIONAL SEARCH REPORT

| | International application No. |
|---|---|
| | **PCT/CN2021/133436** |

**A. CLASSIFICATION OF SUBJECT MATTER**

A61B 5/08(2006.01)i

According to International Patent Classification (IPC) or to both national classification and IPC

**B. FIELDS SEARCHED**

Minimum documentation searched (classification system followed by classification symbols)

A61B 5

Documentation searched other than minimum documentation to the extent that such documents are included in the fields searched

Electronic data base consulted during the international search (name of data base and, where practicable, search terms used)

CNABS, CNTXT, VEN, DWPI: 光电容积, 脉搏波, 呼吸, 间期, 间隔, 峰, 异常, 矫正, 矫正, 滤波, ppg, photoplethysmograph, respiratory, rate, filter, waveform, interval , sequence

**C. DOCUMENTS CONSIDERED TO BE RELEVANT**

| Category* | Citation of document, with indication, where appropriate, of the relevant passages | Relevant to claim No. |
|---|---|---|
| X | US 2019117097 A1 (HILL ROM SERVICES PTE LTD.) 25 April 2019 (2019-04-25) description, paragraphs 41-57, and figures 1-15 | 1-14 |
| A | CN 106777884 A (BEIJING XINLIANG SCIENCE AND TECHNOLOGY CO., LTD.) 31 May 2017 (2017-05-31) entire document | 1-14 |
| A | US 2019133537 A1 (TATA CONSULTANCY SERVICES LTD.) 09 May 2019 (2019-05-09) entire document | 1-14 |
| A | CN 106539586 A (GUANGZHOU CVTE ELECTRONIC TECHNOLOGY CO., LTD.) 29 March 2017 (2017-03-29) entire document | 1-14 |
| A | CN 112494031 A (XIANNING VOCATIONAL TECHNICAL COLLEGE; WUHAN ZONCARE BIO MEDICAL ELECTRONICS CO., LTD.) 16 March 2021 (2021-03-16) entire document | 1-14 |
| A | CN 112494008 A (SHENZHEN FENDA INTELLIGENT TECHNOLOGY CO., LTD.) 16 March 2021 (2021-03-16) entire document | 1-14 |

☑ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **19 February 2022** | **02 March 2022** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/133436** |

### C. DOCUMENTS CONSIDERED TO BE RELEVANT

| Category* | Citation of document, with indication, where appropriate, of the relevant passages | Relevant to claim No. |
|---|---|---|
| A | CN 105662345 A (SHENZHEN H&T INTELLIGENT CONTROL CO., LTD.) 15 June 2016 (2016-06-15)<br>entire document | 1-14 |
| A | CN 101732050 A (XI'AN JIAOTONG UNIVERSITY) 16 June 2010 (2010-06-16)<br>entire document | 1-14 |
| A | WO 0021438 A1 (UNIV FLORIDA) 20 April 2000 (2000-04-20)<br>entire document | 1-14 |
| A | KR 101922221 B1 (PUSAN NATIONAL UNIVERSITY INDUSTRIAL COOPERATION FOUND) 26 November 2018 (2018-11-26)<br>entire document | 1-14 |

Form PCT/ISA/210 (second sheet) (January 2015)

**INTERNATIONAL SEARCH REPORT**
Information on patent family members

International application No.

**PCT/CN2021/133436**

| Patent document cited in search report | | | Publication date (day/month/year) | Patent family member(s) | | | Publication date (day/month/year) |
|---|---|---|---|---|---|---|---|
| US | 2019117097 | A1 | 25 April 2019 | US | 10993630 | B2 | 04 May 2021 |
| | | | | EP | 3473169 | A1 | 24 April 2019 |
| CN | 106777884 | A | 31 May 2017 | None | | | |
| US | 2019133537 | A1 | 09 May 2019 | EP | 3479758 | A1 | 08 May 2019 |
| CN | 106539586 | A | 29 March 2017 | None | | | |
| CN | 112494031 | A | 16 March 2021 | None | | | |
| CN | 112494008 | A | 16 March 2021 | None | | | |
| CN | 105662345 | A | 15 June 2016 | WO | 2017118127 | A1 | 13 July 2017 |
| CN | 101732050 | A | 16 June 2010 | CN | 101732050 | B | 01 February 2012 |
| WO | 0021438 | A1 | 20 April 2000 | WO | 0021438 | A9 | 31 August 2000 |
| | | | | AU | 1118500 | A | 01 May 2000 |
| KR | 101922221 | B1 | 26 November 2018 | None | | | |

Form PCT/ISA/210 (patent family annex) (January 2015)